# EUROPEAN PATENT SPECIFICATION

(11) **EP 1 061 992 B1**
(45) Date of publication and mention of the grant of the patent: **03.11.2004**
(21) Application number: 99910921.8
(22) Date of filing: 15.03.1999
(51) Int. Cl.: A61M 39/28, A61M 25/01

(54) **URINARY CATHETER WITH LOCKING**
BLASENKATHETER MIT VERRIEGELUNG
CATHETER URINAIRE POURVU D'UN DISPOSITIF DE BLOCAGE

(30) Priority: 13.03.1998 SE 9800862
(43) Date of publication of application: 27.12.2000
(73) Proprietor: Radojevic, Borka, 260 40 Viken (SE)
(72) Inventor: Bjurenvall, Ingemar, London W1X 7HB (GB)
(74) Representative: Norén, Per Bo Arne
(86) International application number: PCT/SE1999/000388
(87) International publication number: WO 1999/045997

(56) References cited:
- SE-B- 465 999
- SE-B- 502 620
- US-A- 2 002 835
- US-A- 4 205 819
- US-A- 4 611 785

## Description

The present invention relates to a urinary catheter, especially for the periodical emptying of the urinary bladder of women, but also of men, and comprising a tubular catheter body having an essentially circular outer crosscut and produced from an inert polymeric material, wherein the catheter body along its length exhibits two flexible thickenings radially projecting from the catheter body and its circumference intended to bear against respective end of urethra, and exhibits one bead arranged between those thickenings, wherein one of the thickenings is displaceble arranged to the catheter body.

The object with the present invention is to provide an improved urinary catheter for preferably periodical emptying of the urinary bladder.

### Background of the invention

From SE-B-465 999 it is previously known a urinary catheter comprising a tubular catheter body having essentially circular outer cross-section and which is made of a polymeric material, wherein the catheter body along its length preferably exhibits two flexible thickenings radially projecting from the catheter body and its circumference intended to bear against respective end of the urethrea, and exhibits a bead arranged between those thickenings. This catheter is intended to be connected to a collecting container for urine in the shape of a plastic bag commonly used for the collection of urine via a catheter.

Further, a urinary catheter is previously known from SE-B-502 620 comprising a tubular catheter body having an essentially outer cross-section and which is made of a polymeric material, wherein the catheter body along its length preferably exhibits two flexible thickenings radially projecting from the catheter body and its circumference intended to bear against respective end of the urethrea, and exhibits a bead arranged between those thickenings, wherein one of the thickenings is displaceble arranged to the catheter body, wherein this later catheter is characterized in that it contains a conductive material.

From the later urinary catheter according to SE-B-502 620 it is known that one of the thickenings along the catheter body is displaceble arranged along said catheter body, to adapt the length of the catheter to the real length of the urethrea, in intention to lock the catheter and prevent irritation. Hereby is no locking of said thickening present, since one rely on the mutual friction of the materials, which, however, is strongly reduced in the environment in which they are present.

### Description of the present invention

It has now been shown possible to solve this problem in an effective way by means of the present invention which is characterized in, that in connection to the displaceble thickening, a locking mechanism is displaceble arranged around the catheter body, wherein the locking mechanism comprises a body having partly a cylindrical, through hole carrying a first part of the catheter body, partly a slit arranged to carry a second part of the catheter body formed by bending of said catheter body.

Further characteristics will appear in the accompanying claims.

By the present invention is achieved partly that an effective locking of the displaceble thickening is achieved, partly that the catheter tube is effectively closed to eliminate leakage there from.

The invention is closer described with reference to the enclosed drawings, where:

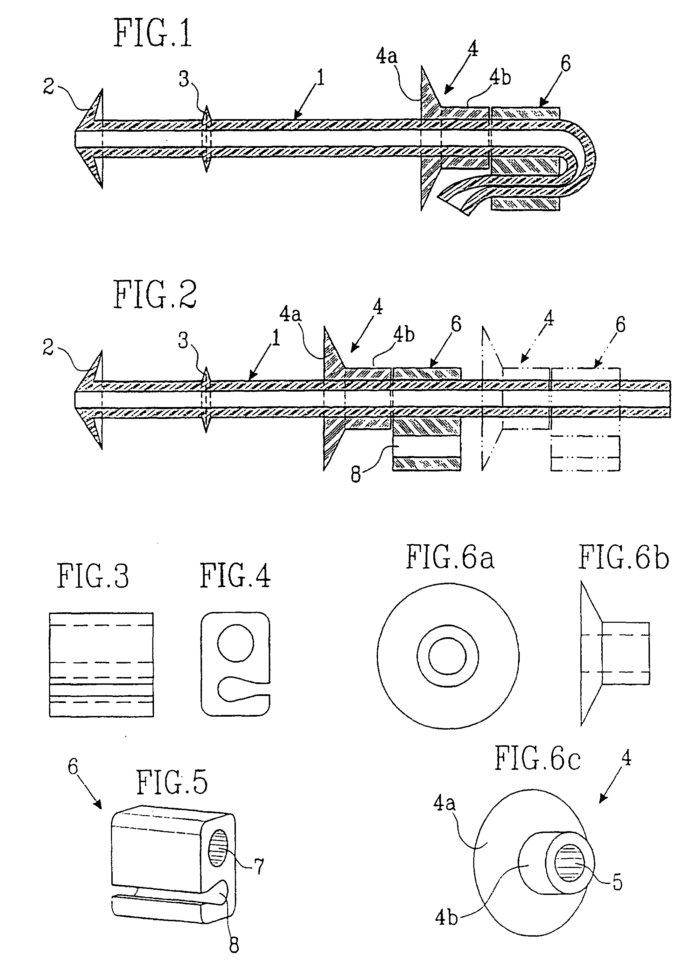

- Fig. 1: shows a side view of a catheter according to the invention in a non-closed state;
- Fig. 2: shows a side view according to Fig. 1 in a closed state;
- Fig. 3: shows a side view of a locking mechanism;
- Fig. 4: shows a cross-section along the line IV - IV in Fig. 3 of the locking mechanism;
- Fig. 5: shows the locking mechanism according to Fig. 3 in perspective; and
- Fig. 6: shows the rear thickening, partly seen from behind (6a), partly from the side (6b), partly in perspective.

A catheter body denoted 1, which essentially comprises a tube of a smooth, flexible polymeric material, such as silicon polymer or the like. The catheter 1 has an outer diameter of 4-5 mm and an inner diameter of 2,5 -3 mm. At the front end of the catheter body 1a thickening 2 is arranged as a parachute-shaped flange having an outer diameter of 15 -18 mm. At a distance from this front end, a collar-shaped bead 3 is present, having a somewhat smaller diameter than the front flange, viz. 7 - 9 mm. On the side facing the flange 2 at the bead 3, an additional thickening 4 is arranged, which thickening having essentially the same outer diameter as the front flange 2, and is displacebly arranged along the catheter body. Thus, this rear thickening 4 is movable along the whole catheter body 1 and exhibits for this purpose partly the flange 4a as such, partly an attachment shaped as a cylindrical body 4b having a inner tube-shaped hole 5 adapted to the outer diameter of the catheter body.

Further, on the catheter body 1 there is arranged a locking mechanism 6 comprising a locking body having a central inner tube-shaped hole 7 and a slit 8 arranged in such way that the cutting plane of the slit 8 does not cut the central hole 7. The central hole 7 has a diameter for the catheter body to fit into it and the locking mechanism can be displaced along the catheter body I with a close fit. The slit 8 has such width that an inserted part of the catheter body 1 closes by means of clamping the inner tube.

The catheter works according to the following:
The front end of the catheter 1 is inserted through urethrea to the urinary bladder where the front flange 2 is allowed to expand and form a sealing and a holder-on against the walls of the urinary bladder. The object of the bead 3, which is placed at a distance of 10-14 mm from the front end of the catheter 1, is to stimulate the closing muscle, the sphincter, to increase its function. The rear flange 4 is inserted towards the lower opening, which for women means about 25 -50 mm from the urinary bladder, and considerably more for men, to secure the catheter in the urethrea, that the catheter does not move along urethrea and thereby creates an irritation and a possible bacterial transport up towards the urinary bladder. The locking mechanism 6 is inserted up against the rear flange 4 as to lock the catheter body with the flanges in position, whereupon the remaining length of the catheter tube is bent upwards and is inserted into the slit 8 to a total locking and closing of the catheter. In connection herewith the catheter is cut to such a length that it only passes to the rear flange 4. When the urinary bladder is full, the catheter is easily released from the locking mechanism and the bladder is emptied. In this way, the catheter does not have to be changed too often, which in itself is a risk, and the urinary bladder is trained together with the sphincter.

The slit 8 may be placed in another direction, wherein the shown embodiment is still preferred since it involves folding in one plane and insertion into the slit in another plane. The hole 7 in the locking mechanism may likewise be designed with a slit opening to the side of the body.

## Claims

1. An urinary catheter, in particular for periodical emptying of the urinary bladder of women, but also of men, and comprising a tubular catheter body (1) having an essentially circular outer cross-section and being produced from an inert polymeric material, wherein the catheter body (1) along its length exhibits two flexible thickenings (2, 4) radially projecting from the catheter body (1) and its circumference intended to bear against the respective end of urethra, and exhibits one bead (3) arranged between those thickenings (2, 4), wherein one of the thickenings (4) is displaceble arranged to the catheter body (1),
**characterized in,**
**that** in connection to the displaceble thickening (4), a locking mechanism (6) is displaceble arranged around the catheter body (1), wherein the locking mechanism (6) comprises a body having partly a cylindrical, through hole (7) carrying a first part of the catheter body (1), partly a slit (8) arranged to carry a second part of the catheter body (1) formed by bending of said catheter body (1).

2. An urinary catheter according to claim 1,
**characterized in,**
**that** the slit (8) extends parallel to the through hole (7).

3. An urinary catheter according to claim 1 - 2,
**characterized in,**
**that** the direction of the slit (8) is such that a plane through the opening of the slit (8) does not cut said through hole (7).

## Patentansprüche

1. Ein Blasenkatheter, insbesondere zum regelmäßigen Entleeren der Harnblase von Frauen, aber auch von Männern, der einen schlauchförmigen Katheterkörper (1) aufweist, der einen im wesentlichen kreisförmigen Außenquerschnitt aufweist und aus einem inerten polymeren Material hergestellt ist, wobei der Katheterkörper (1) entlang seiner Länge zwei flexible Verdickungen (2, 4) aufweist, die sich von dem Katheterkörper (1) radial erstrecken, und deren Umfang gegen das jeweilige Harnröhrenende drücken soll, und der einen zwischen diesen Verdickungen (2, 4) angeordneten Wulst (3) aufweist, wobei eine der Verdickungen (4) verschiebbar an dem Katheterkörper (1) angeordnet ist,
**dadurch gekennzeichnet,**
**dass** in Verbindung mit der verschiebbaren Verdickung (4) ein Verriegelungsmechanismus (6) um den Katheterkörper (1) verschiebbar angeordnet ist, wobei der Verriegelungsmechanismus (6) einen Körper aufweist, der teils ein zylindrisches Durchgangsloch (7), das einen ersten Teil des Katheterkörpers (1) trägt, teils einen Schlitz (8) aufweist, der angeordnet ist, um einen zweiten Teil des Katheterkörpers (1) zu tragen, der durch Biegen des Katheterkörpers (1) gebildet wird.

2. Ein Blasenkatheter gemäß Anspruch 1,
**dadurch gekennzeichnet,**
**dass** sich der Schlitz (8) parallel zu dem Durchgangsloch (7) erstreckt.

3. Ein Blasenkatheter gemäß Anspruch 1 oder 2,
**dadurch gekennzeichnet,**
**dass** die Richtung des Schlitzes (8) derart ist, dass eine Ebene durch die Öffnung des Schlitzes (8) nicht das Durchgangsloch (7) schneidet.

## Revendications

1. Cathéter urinaire, particulièrement destiné à vider périodiquement la vessie urinaire de femmes, mais aussi d'hommes, et comprenant un corps de cathéter (1) tubulaire possédant une section droite externe sensiblement circulaire et étant réalisé en une matière plastique polymère inerte, où le corps de cathéter (1) présente, sur sa longueur, deux épaississements souples (2, 4) faisant radialement saillie du corps de cathéter (1) et sa circonférence destinée à porter contre l'extrémité respective de l'urètre, et il présente un bourrelet (3) unique disposé entre les épaississements (2, 4), où l'un des épaississements (4) est disposé de manière à pouvoir être déplacé par rapport au corps de cathéter (1),
**caractérisé en ce que**, en liaison avec l'épaississement déplaçable (4), un mécanisme de blocage (6) est disposé, de manière à pouvoir être déplacé, autour du corps de cathéter (1), où le mécanisme de blocage (6) comprend un corps possédant, pour partie, un trou traversant cylindrique (7) portant une première partie du corps de cathéter (1), et, pour partie, une fente (8) disposée de manière à porter une deuxième partie du corps de cathéter (1) formée par flexion dudit corps de cathéter (1).

2. Cathéter urinaire selon la revendication 1, **caractérisé en ce que** la fente (8) s'étend parallèlement au trou traversant (7).

3. Cathéter urinaire selon la revendication 1 ou 2, **caractérisé en ce que** la direction de la fente (8) est telle qu'un plan passant par l'ouverture de la fente (8) ne coupe pas ledit trou traversant (7).
